# EUROPEAN PATENT APPLICATION

(11) **EP 2 589 361 A1**
(43) Date of publication of application: **08.05.2013**
(21) Application number: 11800148.6
(22) Date of filing: 24.06.2011
(51) Int. Cl.: A61G 5/00, A61F 5/44

(54) **AUTOMATIC WHEELCHAIR-TYPE FECES CLEANER WITHOUT NEED OF UNDRESSING AND DRESSING**

(30) Priority: 29.06.2010 CN 201010217657
(71) Applicant: Huang, Chunming, Nanan, Fujian 362000 (CN)
(72) Inventor: Huang, Chunming, Nanan, Fujian 362000 (CN)
(74) Representative: Chaillot, Geneviève
(86) International application number: PCT/CN2011/076240
(87) International publication number: WO 2012/000403

(57) **Abstract**

An automatic wheelchair-type feces cleaner without need of undressing and dressing comprises a wheelchair (1). A fully automatic treater (2) and adaptive pants (3) are provided on the wheelchair (1). The fully automatic treater (2) has an excretion duct (213), an excrement collection device (21), a negative pressure device (22), a positive pressure device (23), a hot water device (24), a hot air device (25) and a control device (26). The adaptive pants (3) are provided with an excrement collection region (33) at the crotch, which is provided with an excretion port (34), an inflating pipe (35), a hot water delivery pipe and a hot air delivery pipe. The inlet end of the excretion duct (213) is connected with the excretion port (34) of the collection region (33), and the outlet end of the excretion duct (213) is connected with the collection device (21) which is connected with the negative pressure device (22). The positive pressure device (23), the hot water device (24) and the hot air device (25) are correspondingly connected with the inflating pipe (35), the hot water delivery pipe and the hot air delivery pipe of the collection region (33) via a piping, respectively. With the automatic wheelchair-type feces cleaner without need of undressing and dressing, a user can defecate at any time, which largely expands the range of movement, and the user can defecate and clean up without the help from others.

## Description

### Technical Field

The present invention relates to the field of personal and household sanitation device, and more particularly to a wheelchair-type excrement automatic cleaning apparatus without need of undressing and dressing which is designed for the mobility disabled person.

### Background Art

Currently put into the market is an excrement and urine automatic disposal system, which is designed for the mobility disabled person and serves to automatically dispose the excrement and urine of the mobility disabled person. The excrement and urine automatic disposal system generally comprises an excrement and urine automatic disposal apparatus and a toilet cooperating with the excrement and urine automatic disposal apparatus. The excrement and urine automatic disposal apparatus is generally provided with an evacuation barrel, a negative pressure device, a hot water device, a hot air device and a control device. The evacuation barrel accommodates excrement and urine discharged from the human body, and is connected with an excrement and urine container via a valve. The excrement and urine container is used to pack and dispose excrement and urine discharged from the evacuation barrel. The toilet is generally provided with an excrement collecting area, a hot water output pipe and a hot air output pipe. The excrement collecting area is further provided with an excrement and urine discharging port which is connected with an excrement and urine excretion duct. The excrement and urine excretion duct is connected with the evacuation barrel via the negative pressure device, for drawing the excrement and urine into the evacuation barrel by negative pressure. The hot water output pipe is connected with the hot water device via a hot water pipe, for flushing the excrement and urine place of a user. The hot air output pipe is connected with the hot air device via a hot air pipe, for drying the excrement and urine place of the user. The control device comprises an interactive interface, which is provided with an ON/OFF switch. In particular, the control device may further be provided with a remote controller for switching ON/OFF.

However, the existing toilets for cooperating with the excrement and urine automatic disposal apparatus have a common feature in that, when the mobility disabled person needs defecation, it is necessary for the user to have a self-care ability or a caregiver to install various toilets as mentioned above to the excrement and urine discharging place; and when the defecation is complete, it is also necessary for the caregiver to remove the toilets. It is impossible to automatically and intelligently discharge the excrement and urine without help from others. Especially due to the operation mode, material and structure of the existing toilet, it can not keep contact with the human body for a long time, and can only be removed from the excrement and urine discharging place by means of a mechanical transmission means or manually by the caregiver. It is relatively inconvenient for use. Besides, when the mobility disabled person goes out, it is impossible to use the toilet at any time and anywhere, which greatly limits the user from going out.

### Disclosure of the Invention

In view of this, it is an object of the present invention to provide a wheelchair-type excrement automatic cleaning apparatus without need of undressing and dressing, which enables the user to defecate at any time and anywhere during going out and largely expands the range of movement. Besides, there is no need for tedious adjustment before and after use, the automatic cleaning without need of undressing and dressing is very convenient for use, and the user does not need help from others during defecate and cleaning up.

The following solutions are proposed in the present invention to achieve the above objects.

A wheelchair-type excrement automatic cleaning apparatus without need of undressing and dressing comprises a wheelchair. The wheelchair is provided with a fully automatic disposal apparatus and self-adapting pants made of a soft facing material. The fully automatic disposal apparatus has an excretion duct, an excrement collecting device, a negative pressure device, a positive pressure device, a hot water device, a hot air device and a control device. The self-adapting pants are provided with an excrement collecting area at the crotch, and the excrement collecting area is provided with an excrement port corresponding to the discharging place of the human body, an inflating pipe, a hot water output pipe and a hot air output pipe. The excretion duct has an inlet end and an outlet end. The inlet end of the excretion duct is connected with the excrement port of the excrement collecting, and the outlet end of the excretion duct is connected with the excrement collecting device. The excrement collecting device is connected with the negative pressure device. The positive pressure device, the hot water device and the hot air device are correspondingly connected with the inflating pipe, the hot water output pipe and the hot air output pipe of the excrement collecting area via a pipe. The control device controls the operation of the fully automatic disposal apparatus and the self-adapting pants.

The cushion of the wheelchair is provided with an excrement port corresponding to the excrement port of the self-adapting pants; the negative pressure device, the positive pressure device, the hot water device and the hot air device are provided on the chair back of the wheelchair; and the excrement collecting device is provided below the cushion of the wheelchair.

The excrement port of the cushion is provided with a cover which can open and close, and the control device controls the operation of the cover.

The excrement collecting device is detachably connected with the wheelchair to form a removable connection.

The excrement collecting device comprises an excrement container which has an airtight hollow chamber. The hollow chamber is divided into an excrement collecting chamber and a negative pressure chamber by a partition plate. The partition plate is provided with a vent hole for communicating with the excrement collecting chamber and the negative pressure chamber. The excrement container is provided with openings, which correspond to the excrement collecting chamber and the negative pressure chamber and are arranged in a staggered manner. The opening of the excrement collecting chamber communicates with the outlet end of the excretion duct, and the opening of the negative pressure chamber communicates with the negative pressure device.

The self-adapting pants are provided with a buttock bump at the rear inner surface of the excrement collecting area.

The self-adapting pants are provided with a convex tube at the front outer surface of the excrement collecting area, and the inflating pipe, the hot water output pipe and the hot air output pipe are arranged in the convex tube, respectively.

The self-adapting pants are provided with compatible sticking parts on the sides, and the excrement port of the self-adapting pants is provided with a quick connector compatible with the inlet end of the excretion duct.

The waistline and nozzle of self-adapting pants are provided with a pneumatic sealing ring, respectively, and the pneumatic sealing ring is connected with an inflating device via a pipe.

The control device comprises an interactive unit which is connected with the wheelchair in a flip and folding manner to form a removable connection.

With the above-mentioned configuration, the wheelchair-type excrement automatic cleaning apparatus without need of undressing and dressing of the present invention has the following beneficial effects.

Firstly, when the user wears the self-adapting pants and trousers and is seated in the wheelchair, he can manipulate the wheelchair by himself or the wheelchair can be manipulated by others to freely move about, which largely expands the range of movement. During defecation, the excretion duct of self-adapting pants passes through the opening at the crotch of trousers to communicate with the excrement collecting device. In this way, the user may be seated in the wheelchair and start the control device, so as to complete the process of defecation and automatic cleaning up. It is not necessary to undress the self-adapting pants and trousers, so that it is not only possible to eliminate the tedious adjustment before and after use, but also to free the caregiver from the tedious cleaning up process on the human body discharging place. After excrement and urine discharged by the human body is flushed to the excrement collecting device, it is only necessary for the caregiver to dispose the excrement, urine and waste water in the excrement collecting device, which can largely improve working conditions of the caregiver and facilitate carrying out the nursing services. By using self-adapting pants made of a soft facing material as the toilet, and designing appropriately the self-adapting pants, such a toilet in the form of pants not only has various functions of the existing toilet, but also is soft and comfortable, so that it can be worn on a user for a long time, and substantially make no difference from the ordinary pants in respect of comfort, wearing process and changing time. During excrement and urine, the user does not need help from others to dress and undress the toilet, and can start the human body excrement disposal apparatus at any time by himself to defecate, so that it is very convenient for use. The moisture generated in the self-adapting pants during hot air drying is drawn into the excrement collecting device by the negative pressure device, which decreases the time for drying. In addition, the moisture is substantially not removed through the human body, and this provides the passage for discharging the moisture, thus saving energy and increasing the comfort for the human body.

Secondly, the self-adapting pants are provided with a buttock bump at the rear inner surface of the excrement collecting area, so that when the user wears the self-adapting pants and sits down, the buttock bump is clamped in the buttock cleavage, thus pushing the left and right buttocks to both sides and preparing for defecation. The excrement collecting area is provided with a convex tube at the front outer surface, and the excrement port is provided with a quick connector, wherein the convex tube and the quick connector can be connected with respective pipe of the fully automatic disposal apparatus via a pipe, so that it is possible to complete the automatical disposal process of defecating and cleaning up, and there is no need for tedious adjustments from medical workers or caregivers before and after use, thus making it very convenient for use. In addition, the self-adapting pants have a simple and reasonable structure, and the convex tube is provided at the outer surface of the self-adapting pants, so that it will not bring discomfort to the human body, and it is also possible to form self-adapting pants as thin as the common pants, thus making it comfortable and fit to wear.

Thirdly, the self-adapting pants are made as movable pants which can be opened or closed on both sides, and thus are more convenient for dressing and undressing. The excrement port of the self-adapting pants is provided with a quick connector at the outer surface, and it has a simple and reasonable structure, so that the user can wear the self-adapting pants for a long time. The quick connector is inserted into the inlet end of the excretion duct by passing through the opening at the crotch of the trousers, so that during cleaning up the defecation it is possible to not only form a sealing connection, but also avoid the tedious adjustments like dressing and undressing, which decreases the user's sense of inconvenience and is very convenient for use. Brief Description of Drawings
Fig. 1 is a first three-dimensional schematic view showing the wheelchair-type excrement automatic cleaning apparatus without need of undressing and dressing of the present invention;
Fig. 2 is a second three-dimensional schematic view showing the wheelchair-type excrement automatic cleaning apparatus without need of undressing and dressing of the present invention;
Fig. 3 is a structural view showing an excrement collecting device;
Fig. 4 is a structural view showing a case body of the excrement collecting device;
Fig. 5 is a structural view showing a negative pressure device, a hot water device and a hot air device;
Fig. 6 is an unfolded schematic view showing a pant body of the self-adapting pants;
Fig. 7 is a top view showing an excrement collecting area of the pant body;
Fig. 8 is a left view showing an excrement collecting area of the pant body;
Fig. 9 is a structural view showing a first embodiment of the of the self-adapting pants;
Fig. 10 is a structural view showing a second embodiment of the self-adapting pants;
Fig. 11 is a schematic view showing the general controlling principle of the wheelchair-type excrement automatic cleaning apparatus without need of undressing and dressing of the present invention;
Fig. 12 is a schematic view showing the process for disposing urine in the wheelchair-type excrement automatic cleaning apparatus without need of undressing and dressing of the present invention; and
Fig. 13 is a schematic view showing the process for disposing excrement in the wheelchair-type excrement automatic cleaning apparatus without need of undressing and dressing of the present invention.

### Best Mode for Carrying out the Invention

To further elucidate the solutions of the present invention, the present invention will hereinafter be set forth in detail by reference to the specific embodiments.

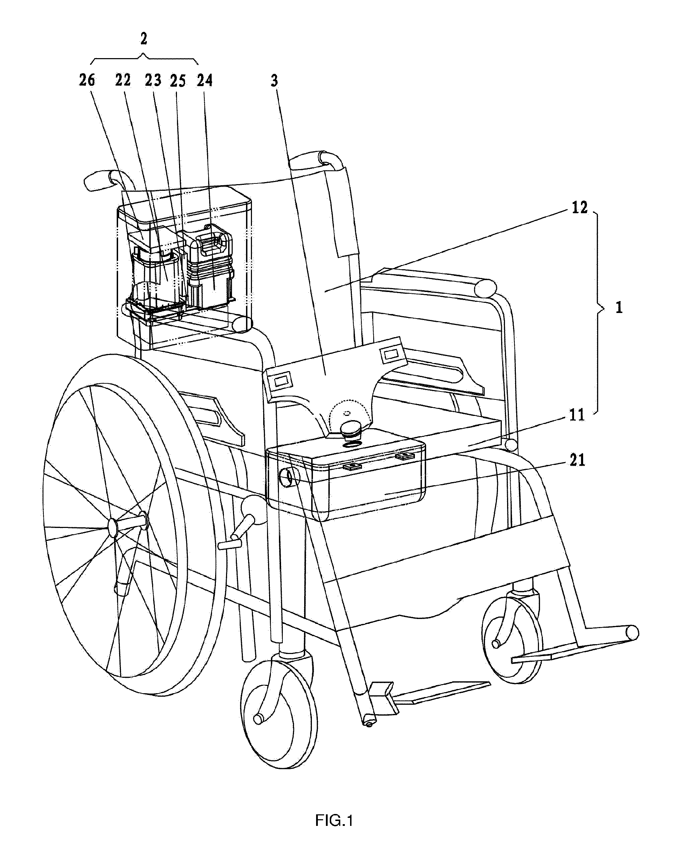

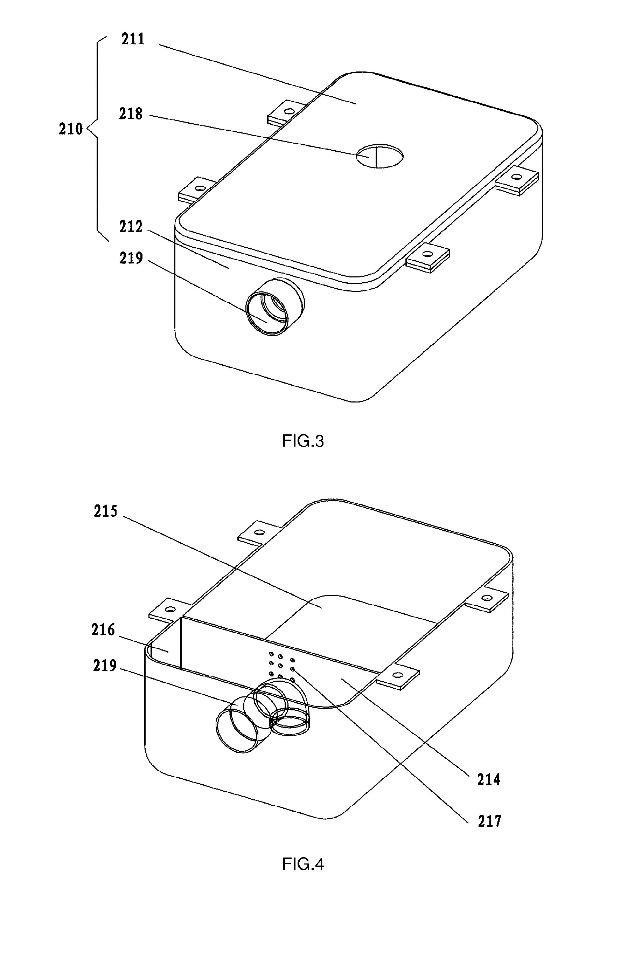

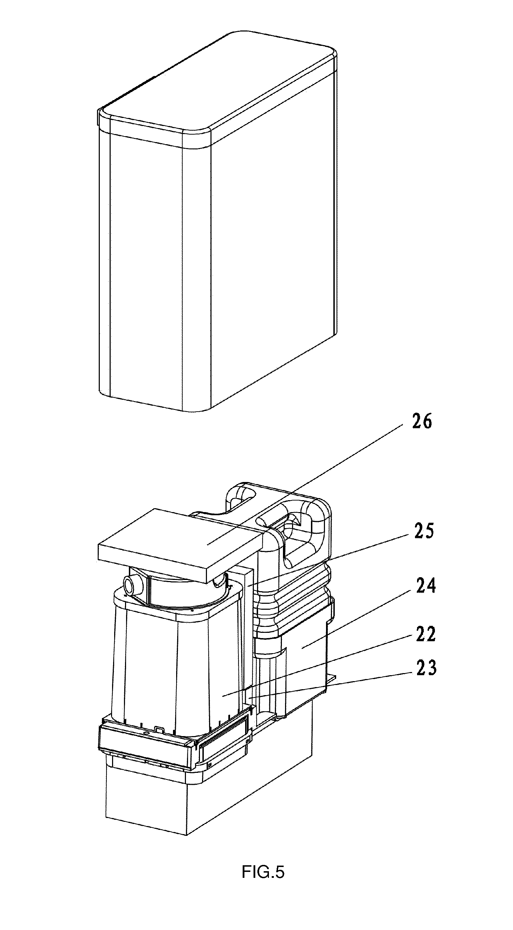

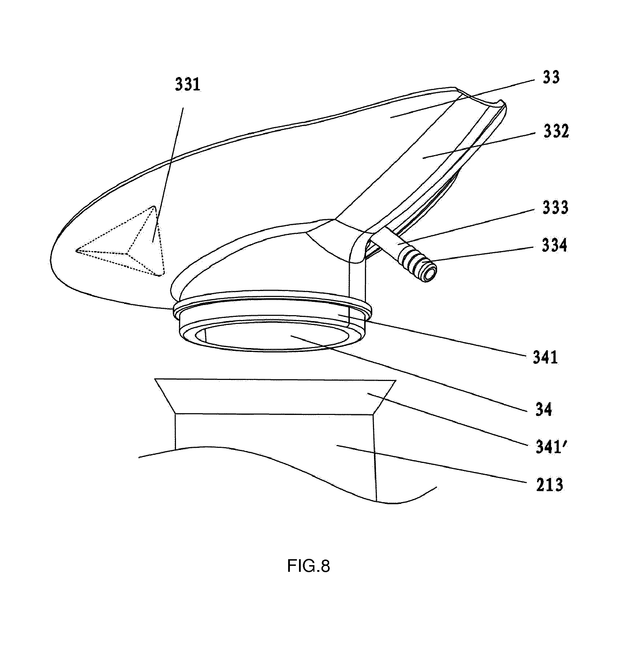

As shown in Figs. 1-13, the wheelchair-type excrement automatic cleaning apparatus without need of undressing and dressing of the present invention comprises a wheelchair 1, a fully automatic disposal apparatus 2 and self-adapting pants 3.

The wheelchair 1 comprises a cushion 11 and a chair back 12, the cushion 11 is provided with an excrement port 111, and the excrement port 111 is provided with a cover. The cover can be opened and closed and manipulated by a control device 26 described later. In particular, the cover is automatically opened and closed according to whether the excrement automatic cleaning apparatus is in service, so as to avoid unpleasant smell. The wheelchair 1 primarily cooperates with the fully automatic disposal apparatus 2 and the self-adapting pants 3, and the remaining structure of the wheelchair 1 is identical to that of the existing wheelchair, which is not described herein for simplicity.

The fully automatic disposal apparatus 2 comprises an excrement collecting device 21, a negative pressure device 22, a positive pressure device 23, a hot water device 24, a hot air device 25 and a control device 26. The excrement collecting device 21 is provided below the cushion 11; and the negative pressure device 22, the positive pressure device 23, the hot water device 24 and the hot air device 25 are provided behind the chair back 12.

The excrement collecting device 21 comprises an excrement container 210 and an excretion duct 213, and the excrement container 210 comprises a cover plate 211 and a case body 212. The case body 212 has a hollow chamber. The hollow chamber is divided into an excrement collecting chamber 215 and a negative pressure chamber 216 by a partition plate 214. The partition plate 214 is provided with a vent hole 217 for communicating with the excrement collecting chamber 215 and the negative pressure chamber 216. The cover plate 211 cooperates with the case body 212 in a sealing manner. The corresponding excrement collecting chamber 215 over the cover plate 211 is provided with an excrement port 218. The excrement port 218 forms a movable connection with the quick connector at the outlet end of the excretion duct 213 via a quick connector. The inlet end of the excretion duct 213 is provided with a quick connector 341'. The quick connector 341' forms a movable connection with the quick connector 341 at the excrement port 34 of the self-adapting pants 3, so that it is convenient to detach the excrement container 210 for cleaning up. At the upper portion of the left chamber wall, the negative pressure chamber 216 is provided with an opening which is provided with a quick connector 219. The quick connector 219 has a port which is located inside the negative pressure chamber 216 and bends downward, so that the port of the quick connector 219 outside the negative pressure chamber 216 is over the port inside the negative pressure chamber 216, and the two ports are arranged in a staggered manner. The quick connector 219 is connected with the quick connector provided at the port of the negative pressure device 22. Of course, the quick connector 219 may also be provided at the chamber wall at the front and rear sides of the negative pressure chamber 216, and the specific installing position can be determined by the port of the negative pressure device 22. The excrement container 210 is connected with the wheelchair 1 in a snapping or sliding manner to form a removable connection. The excrement container 210 is connected with the self-adapting pants 3 via the excretion duct 213, to receive excrement and urine discharged by the human body. Of course, the excrement container 210 further receives the waste water after flushing the user's excrement and urine by the hot water device 24. After the user completes defecation, the excrement container 210 may be removed from the wheelchair 1 for cleaning up. The inner walls of the cover plate 211 and the case body 212 are coated with a layer of anti-adhesive material in order to facilitate cleaning up.

The negative pressure device 22 comprises a vacuum pump. The vacuum pump is connected with the excrement collecting device 21 via a pipe, and serves to form a negative pressure, so as to draw excrement and urine discharged by the human body into the excrement collecting device 21. The pipe for communicating with the excrement collecting device 21 with the self-adapting pants 3 and the pipe for communicating with the excrement collecting device 21 and the negative pressure device 22 are arranged in a staggered manner, in order to prevent excrement and urine in the self-adapting pants 3 from being directly drawn into the negative pressure device 22. In this embodiment, the self-adapting pants 3 communicate with the excrement collecting chamber 215 of the excrement container 210 via the excrement port 34, the excretion duct 213, and the excrement port 218 of the cover plate 211, while the negative pressure device 22 communicates with the negative pressure chamber 216 via its pipe and the opening in a side of the excrement container 210.

As a specific embodiment of the negative pressure device 22, the negative pressure device 22 comprises a vacuum pump 221 and a discharging filter, the vacuum pump 221 is connected with the excrement collecting device 21 via a pipe, and the discharging filter is arranged between the vacuum pump 221 and the excrement collecting device 21. By means of the negative pressure action in the vacuum pump 221, the waste in the self-adapting pants 3 is drawn into the excrement collecting device 21.

The positive pressure device 23 is connected with the self-adapting pants 3 via a pipe, and is used to form a positive pressure to expand the excrement collecting area 33 of the self-adapting pants 3, so that the negative pressure device 22 can draw the waste into the excrement collecting device 21 more smoothly. In particular, as a preferred embodiment of the positive pressure device 23, the positive pressure device 23 comprises an air pump, an air filter, a pressure sensor, an electronically controlled pressure relief valve and a check valve. The air pump communicates with the atmosphere via the air filter, so as to ensure cleanliness of the air. The check valve is arranged in the pipe between the air pump and the self-adapting pants 3 to maintain the air pressure in the self-adapting pants 3. Both the electronically controlled pressure relief valve and the pressure sensor are arranged in the self-adapting pants 3. The electronically controlled pressure relief valve is connected with and controlled by the control device 26. Once the use of the wheelchair-type excrement automatic cleaning apparatus without need of undressing and dressing is complete, the electronically controlled pressure relief valve releases the air pressure in the self-adapting pants 3, and the pressure sensor functions as an overpressure protection.

The hot water device 24 communicates with a hot water output pipe of the self-adapting pants 3 via a pipe, and provides water flow for flushing the user's excrement and urine place. After the water flow from the hot water device 24 flushes the user's excrement and urine place, it directly flows into the excrement collecting chamber 215 of the excrement collecting device 21 via the excretion duct 213. That is, the connection part between the hot water device 24 and the self-adapting pants 3 should be at a higher position. In this embodiment, the hot water device 24 is provided at a higher portion of the chair back 12 of the wheelchair 1, and it specific position is adjusted according to actual requirements, which is not described herein in detail.

In order to keep the excrement collecting device 21 clean, the hot water device 24 further comprises a flushing reversal valve which is provided at the pipe between the water pump and the self-adapting pants 3. The flushing reversal valve is further connected with the excrement collecting device 21 via a pipe. In this way, by means of the flushing reversal valve, it is possible to flush the excrement collecting device 21, so as to improve its cleanliness and avoid bacteria growth.

As a specific embodiment of the hot water device 24, the hot water device 24 comprises a water tank, a water pump, a heating device and a temperature sensor. The water pump, the heating device and the temperature sensor are all provided in the water tank. The heating device heats the water in the water tank, and keeps at a constant temperature with the aid of the temperature sensor. The water pump further communicates with the hot water output pipe of the self-adapting pants 3 via a pipe. Preferably, there are two water tanks, namely, a large water tank and a small water tank. The large water tank is used to store water added with a disinfectant. The heating device, the temperature sensor and the water pump are all arranged in the small water tank, so that the heating device only needs to thermally insulate the small water tank as desired, so that the power consumption is decreased. Of course, a liquid level sensor may further be provided in the large water tank for monitoring the water level in the large water tank. When the water level is below a lower limit, the liquid level sensor issues an alarm.

The hot air device 25 is connected with the self-adapting pants 3 via a pipe, and produces an air flow for drying the user's excrement and urine place. In particular, after drying the user's excrement and urine place, the air flow produced by the hot air device 25 is discharged via the negative pressure device 22.

As a specific embodiment of the hot air device 25, the hot air device 25 comprises a heat exchanger and an air pump. The air pump is connected with a hot air output pipe of the self-adapting pants 3 via a pipe. The heat exchanger is arranged in the pipe between the air pump and the self-adapting pants 3.

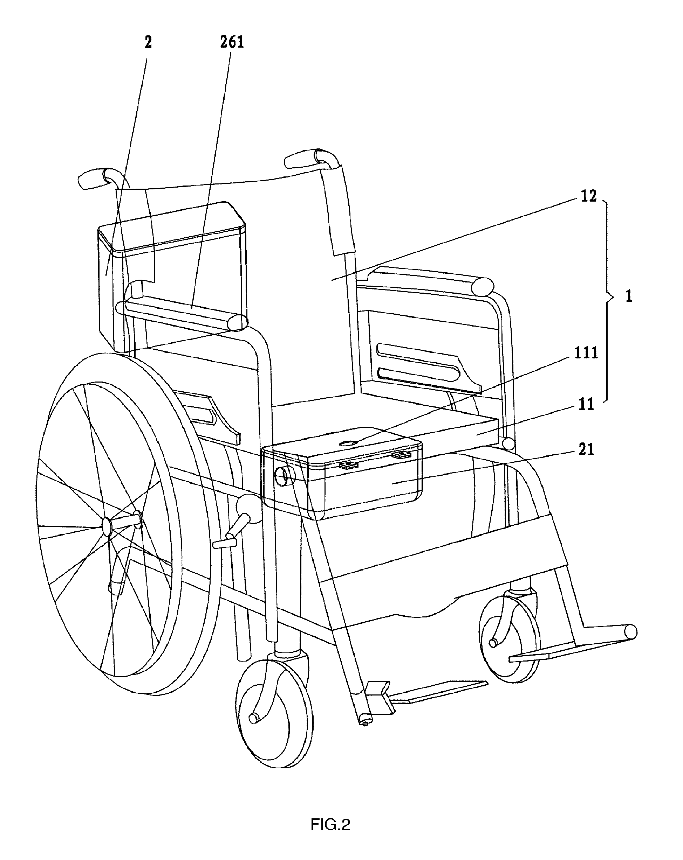

The control device 26 is connected with the negative pressure device 22, the positive pressure device 23, the hot water device 24 and the hot air device 25, and controls the cooperation among them, i.e., controls the operating order of these devices. Of course, the control device 26 may be further provided with a remote control unit as necessary, in order to switch on and off the whole wheelchair-type excrement automatic cleaning apparatus without need of undressing and dressing in a wireless manner. In order to avoid interference from the neighboring apparatus, a dual coding is applied in the remote control signal. Of course, in order to enable the control device 26 to read from each device, an A/D or D/A converting unit is further built in the control device 26 for analog to digital conversion and digital to analog conversion. An interactive unit 261 may be further provided at the armrest of the wheelchair 1 as required, as shown in Fig. 2, which may enable to set and modify the apparatus control parameters, set the operation mode, display the current operation status, and provide manual control buttons or the like. In particular, the interactive unit 261 uses a LCD display with a touch screen, so that the user can manipulate manually. It is noted that the pipe in the present invention can be formed by silicone, latex or the like, and a quick connector may be further arranged between the pipe and the self-adapting pants 3 to improve convenience during assembly and disassembly.

In this way, the wheelchair-type excrement automatic cleaning apparatus without need of undressing and dressing of the present invention performs the following procedures in a sequential and cycling manner under control of the control device 26. Firstly, the positive pressure device 23 expands the excrement collecting area 33 of the self-adapting pants 3 so that the user can defecate. The negative pressure device 22 draws excrement and urine discharged by the user into the excrement collecting chamber 215 of the excrement collecting device 21, then the user's excrement and urine place is flushed with the water flow blown from the hot water device 24, the user's excrement and urine place is dried by the air flow produced by the hot air device 25, and then the excrement container 210 of the excrement collecting device 21 is removed. The caregiver only needs to clean up the excrement container 210. As a result, the present invention can largely improve working conditions in which the caregiver cleans up the excrement collecting device 21, and facilitate carrying out the nursing services. In addition, the moisture generated in the self-adapting pants 3 during hot air drying is drawn into the excrement collecting device 21 by the negative pressure device 22, which decreases the time for drying. In addition, the moisture is substantially not removed through the human body, and this provides the passage for discharging the moisture, thus saving energy and increasing the comfort for the human body.

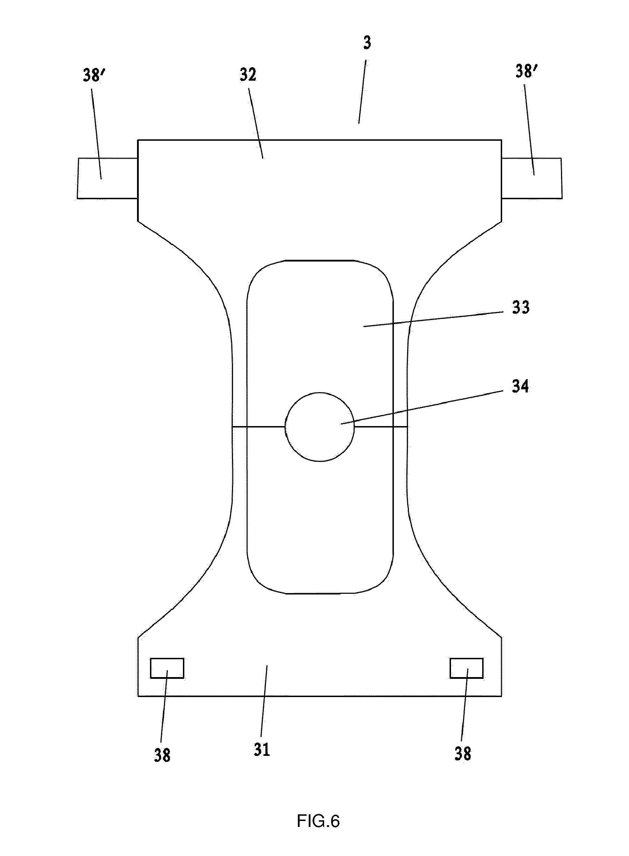

The main part of the self-adapting pants 3 is a pant body 30, as shown in Fig. 6. The facing material of the pant body may be made of an ordinary facing material, like cotton, Terylene, nylon, spandex, Lycra, silk, Modal or the like. In this embodiment, the pant body 30 is movable pants which have a structure similar with diaper. A front piece 31 and a rear piece 32 are provided with respective sticking parts 38, 38' on both sides. The front piece 31 and the rear piece 32 may be stuck to form a triangular or straight angle pant body 30. In a preferred embodiment, the pant body 30 is made of a cotton facing material, which may be treated naturally and is waterproof and air permeable.

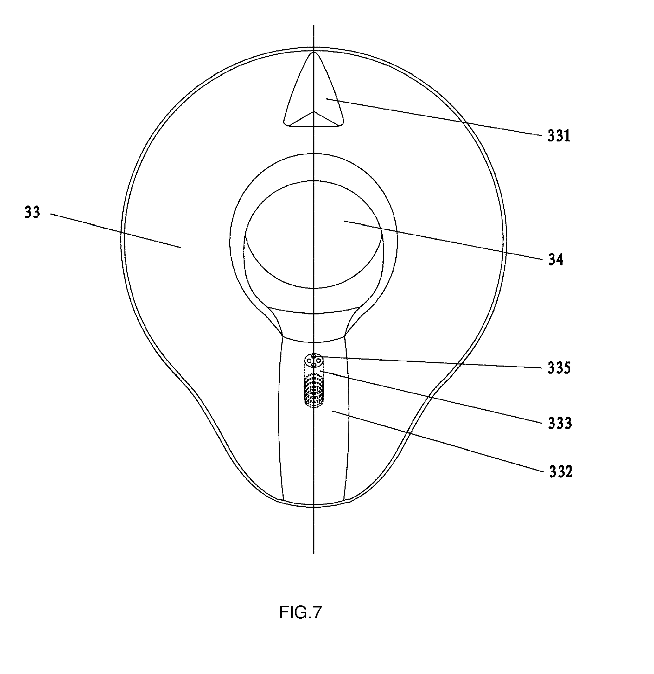

The excrement collecting area 33 is provided at the crotch of the pant body 30 near the excrement and urine discharging port, as shown in Fig. 6. The four corners of the excrement collecting area 33 are rounded angles or elliptical to better fit the body. In another embodiment of the excrement collecting area 33, as shown in Figs. 7-8, the excrement collecting area 33 is elliptical corresponding to the buttocks and has an area tapering from the rear to the front, and an inner surface of the excrement collecting area 33 is a smooth curved surface fit to the human body. The excrement collecting area 33 is provided with a buttock bump 331 made of soft rubber at the rear inner surface. The buttock bump 331 is a triangular bump with smooth edges. When the user wears the self-adapting pants 3 and sits down, the buttock bump 331 is clamped in the buttock cleavage, thus pushing the left and right buttocks to both sides and preparing for defecation. The excrement collecting area 33 is provided with a concave groove 332 at the front. A convex tube 333 is provided at the groove 332 and on the outer surface of the pant body 30. The convex tube 333 is provided with four through-holes, each of which is connected with the respective pipe for the positive pressure device, the disinfection cleaning fluid, the hot water device and the hot air device of the excrement and urine fully automatic disposal apparatus. In this way, the water flow blown from the convex tube 333 may quickly aggregate along the groove 332 after flushing to enter the excrement port 34, instead of spreading around. The convex tube 333 has an inlet end 334 at an outer surface of the pant body 30 and an outlet end 335 at an inner surface of the pant body 30. The outer surface of the inlet end 334 is provided with several stepped grooves for forming a quick and tight connection with the positive pressure device 23, the disinfection cleaning fluid, the hot water device 24, the hot air device 25 or the like. The above mentioned four outlets in the outlet end 335 are arranged to correspond to the human body discharging place.

The excrement port 34 is provided at the bottom of the excrement collecting area 33. The excrement port 34 is provided with a quick connector 341 compatible with the inlet end of the excretion duct 213. The quick connector 341 passes through the excrement port 111 of the cushion 11, and is inserted into the inlet end of the excretion duct 213. Of course, it is possible to arrange the inlet end of the excretion duct 213 and the excrement port 111 of the cushion 11 in a movable connection. The quick connector 341 is made of a soft material like silicone, latex or the like, so that it is very soft, comfortable and less sensible when being contact with the human body. When the user leaves the wheelchair 1 to sit down or lie down, the quick connector substantially has no effect on the human body. The quick connector 341 may be formed in a shape of cone, truncated cone, circle, ellipse, polygon or the like.

The excretion duct 213 is a thick hose made of a soft material like silicone, latex or the like. The thick hose may be connected with the self-adapting pants for a long time, and is soft, comfortable and less sensible when being contact with the human body. The thick hose substantially has no effect on the human body. The inlet end of the excretion duct 213 is provided with another quick connector 341' corresponding to the quick connector 341. The quick connector 341 may be quickly fastened with the quick connector 341', so that the excrement collecting area 33 communicates with the excrement collecting chamber 215 of the excrement container 210 via the excrement port 34 and the excretion duct 213 in sequence.

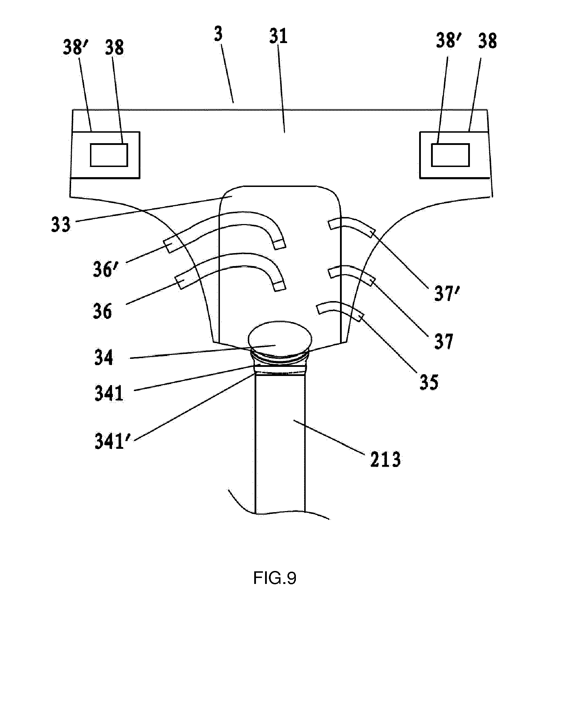

There are two embodiments for the self-adapting pants 3. The first embodiment is shown in Fig. 9. The pant body 30 is further provided with an inflating pipe 35, a hot water output pipe and a hot air output pipe. The hot water output pipe comprises a urine spray pipe 36 and an excrement spray pipe 36'. The hot air output pipe comprises a urine hot air pipe 37 and an excrement hot air pipe 37'. The inflating pipe 35, the urine spray pipe 36, the excrement spray pipe 36', the urine hot air pipe 37 and the excrement hot air pipe 37' are thin hoses made of a soft material like silicone, latex or the like.

The outlet end of the inflating pipe 35 is located in the pant body 30. The inlet end of the inflating pipe 35 is formed by extending the nozzle of the pant body 30 to the outside of the pant body 30, and can be connected with the air outlet of the positive pressure device 23 via a pipe.

The outlet ends of the urine spray pipe 36, the excrement spray pipe 36', the urine hot air pipe 37 and the excrement hot air pipe 37' extend to respective positions in the pant body 30, i.e., corresponding to the position of the excrement port or that of the urine excrement port. The inlet ends of the urine spray pipe 36, the excrement spray pipe 36', the urine hot air pipe 37 and the excrement hot air pipe 37' are formed by extending from the nozzle of the pant body 30 to the outside of the pant body 30, and can be connected with respective pipe of the hot water device 24 and the hot air device 25 of the fully automatic disposal apparatus 2.

The inflating pipe 35, the urine spray pipe 36 and the urine hot air pipe 37 are fixedly arranged in the front piece 31 of the pant body 30. The excrement spray pipe 36' and the excrement hot air pipe 37' are fixedly arranged in the rear piece 32 of the pant body 30. It is also possible to arrange interlayers in the front piece 31 and the rear piece 32 of the pant body 30, and then arrange the inflating pipe 35, the urine spray pipe 36, the excrement spray pipe 36', the urine hot air pipe 37 and the excrement hot air pipe 37' in respective interlayers.

During use, the user may start operation of the wheelchair-type excrement automatic cleaning apparatus without need of undressing and dressing manually or via a wireless remote controller. In addition, it is also possible to set a sensor automatic mode to start the wheelchair-type excrement automatic cleaning apparatus without need of undressing and dressing upon automatically detecting a defecation action. The quick connector 341 of the self-adapting pants 3 passes through the opening at crotch of the trousers and is arranged within the excretion duct 213. The control device 26 opens the above mentioned cover of the excrement port 111 of the cushion 11. The positive pressure device 23 produces an air pressure and inflates the pant body 30 via the inflating pipe 35, so that the excrement collecting area 33 is expanded and falls to form a discharging space for excrement and urine. At this time, the wheelchair-type excrement automatic cleaning apparatus without need of undressing and dressing produces a negative pressure at the excrement port 34 via the negative pressure device 22 and the excretion duct 213. When the user begins to discharge excrement or urine, the discharged excrement or urine is immediately drawn by the negative pressure to the excretion duct 213, and finally falls into the excrement container 210. With the control device 26, the blown hot water is controlled at a predetermined temperature. The hot water is blown to flush the excrement and urine place of the human body via the urine spray pipe 36 and the excrement spray pipe 36'. After flushing, the blown hot air is controlled at a predetermined temperature with the control device 26. The hot air is blown to the excrement place and the urine place via the urine hot air pipe 37 and the excrement hot air pipe 37', so as to dry the excrement place, the urine place, and the whole excrement collecting area 33. The hot air containing moisture is finally drawn by the negative pressure through the excretion duct 213, so as to form a moisture circulation system, which quickly dries the human body and the self-adapting pants.

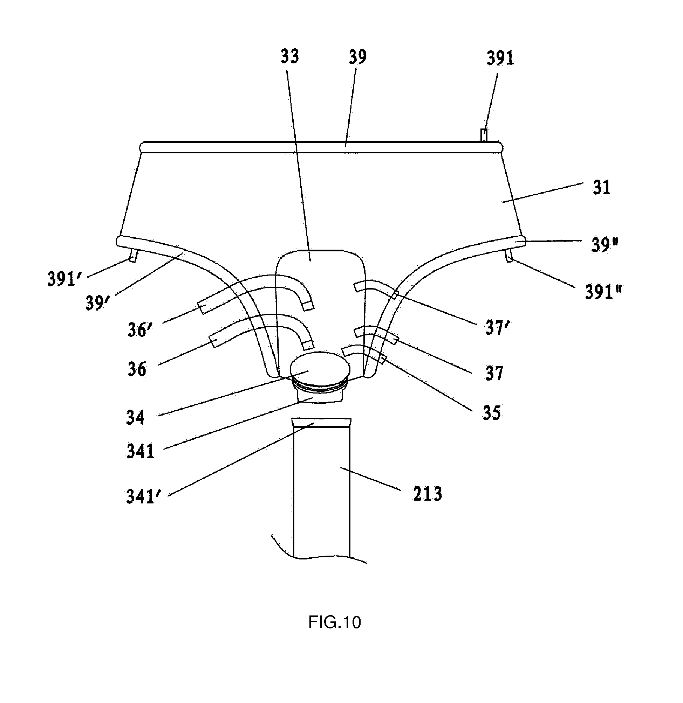

The second embodiment of the self-adapting pants 3 is shown in Fig. 10, which differs from the first embodiment in the structure of the pant body. The pant body 30 may be a triangular pant with a conventional structure, and may also be a four-angle pant. Besides, pneumatic sealing rings 39, 39' and 39" are sewed at the waistline and nozzle of the pant body 30 as sealing parts. The pneumatic sealing rings 39, 39' and 39" have a ring structure sewed by an airtight facing material, and have a certain flexibility to fit to fat or thin human bodies. The pneumatic sealing rings 39, 15 and 16 are provided with inflating ports 391, 391' and 391 ", respectively. The inflating ports 391, 391' and 391 " are connected with the inflating device via a pipe, respectively. During use, the wheelchair-type excrement automatic cleaning apparatus without need of undressing and dressing produces a positive pressure gas via the inflating device .The positive pressure gas inflates the pneumatic sealing rings 39, 39' and 39" via the inflating ports 391, 391' and 391 ". The pneumatic sealing rings 39, 39' and 39" are quickly inflated to expand, thus tightening the waistline and nozzle of the pant body 30 to form a sealing. As a result, when the pant body 30 is inflated by the inflating pipe 35, and the soft and waterproof excrement collecting area 33 is expanded to form a discharging space, it is possible to improve the tightness of the pant body 30, and thus to quickly form a discharging space. In the above-mentioned inflating device, the positive pressure device 23 or the hot air device 25, the positive pressure device 23 or the hot air device 25 may communicate via a pipe with the pneumatic sealing rings 39, 39' and 39" respectively.

The structure of other portions and the operation principle of this embodiment are identical to those of the first embodiment.

In the present invention, the pants with a conventional structure that people have to wear in daily life are designed with a special structure to form self-adapting pants for the mobility disabled person. The mobility disabled person wears the self-adapting pants in daily life. When the user needs to discharge excrement and urine, the quick connector 341 on the bottom of the self-adapting pants 3 communicates with the fully automatic disposal apparatus 2 via the opening at the crotch of trousers. The automatic control system is started via active control of a person (manually or by wireless remote control) or by a sensor which can sense excrement and urine, so that the self-adapting pants automatically converts to a defecation tool for receiving excrement and urine, and there is no need for tedious adjusting process for dressing and undressing the pants/trousers. After the excrement and urine is complete, the automatic control system is started via active control of a person (manually or by wireless remote control) or by a sensor which can sense the excrement and urine, so that the self-adapting pants automatically converts to the common pants which the mobility disabled person have to wear in daily life. The self-adapting pants are usually worn on the human body, and substantially make no difference from the ordinary pants in respect of comfort, wearing process and changing time. During excrement and urine, the user does not need help from others to dress and undress the toilet, and can start the fully automatical disposal of excrement and urine at any time by himself to defecate, so that it is convenient, simple and comfortable to use.

Herein, the inlet ends of the inflating pipe 35, the urine spray pipe 36, the excrement spray pipe 36', the urine hot air pipe 37, the excrement hot air pipe 37', the pneumatic sealing ring 39, the pneumatic sealing ring 39' and the pneumatic sealing ring 39" form quick connections with respective pipe via the compatible quick connectors, so that it is easy to replace the self-adapting pants 3.

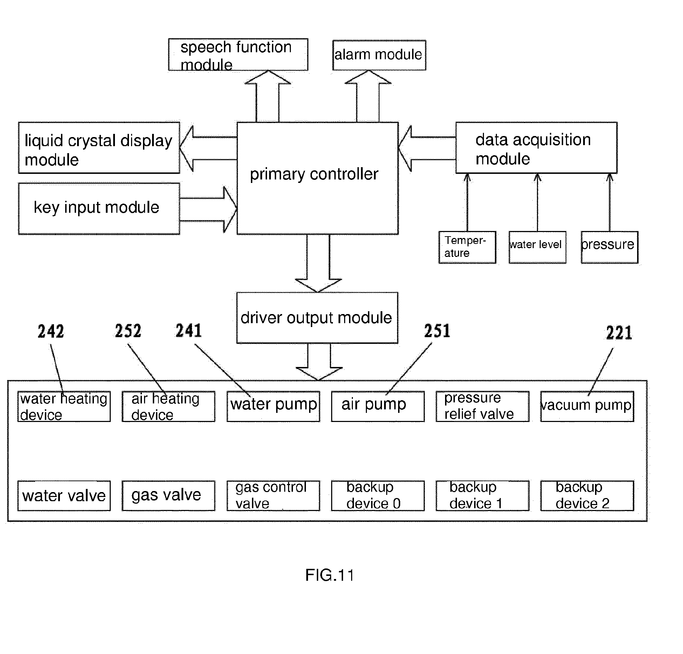

The fully automatic disposal apparatus 2 and the self-adapting pants 3 cooperate with each other, and the control device 26 controls the operation sequence of each device. In particular, as shown in Fig. 11, an input terminal for a data acquisition module of the control device 26 is fed with temperature, water level and pressure signals, and the output terminal of the data acquisition module is connected with respective input terminal of the primary controller. The primary controller is connected with respective ends of the interactive unit via an interactive interface. In this embodiment, a key input module is used. The output terminal of the key input module is connected with respective input terminal of the primary controller, and the output terminal of the key input module is connected with respective input terminal of an alarm module, a speech function module, a liquid crystal display module and a driver output module. The output terminal of the driver output module controls the following devices respectively: the vacuum pump 221 of the negative pressure device 22, the positive pressure device 23, the water pump 241 and the water heating device 242 of the hot water device 24, the air pump 251 and the air heating device 252 of the hot air device 25, as well as a water valve, a gas valve, a gas control valve, a pressure relief valve, a backup device 0, a backup device 1 and a backup device 2 in each pipe.

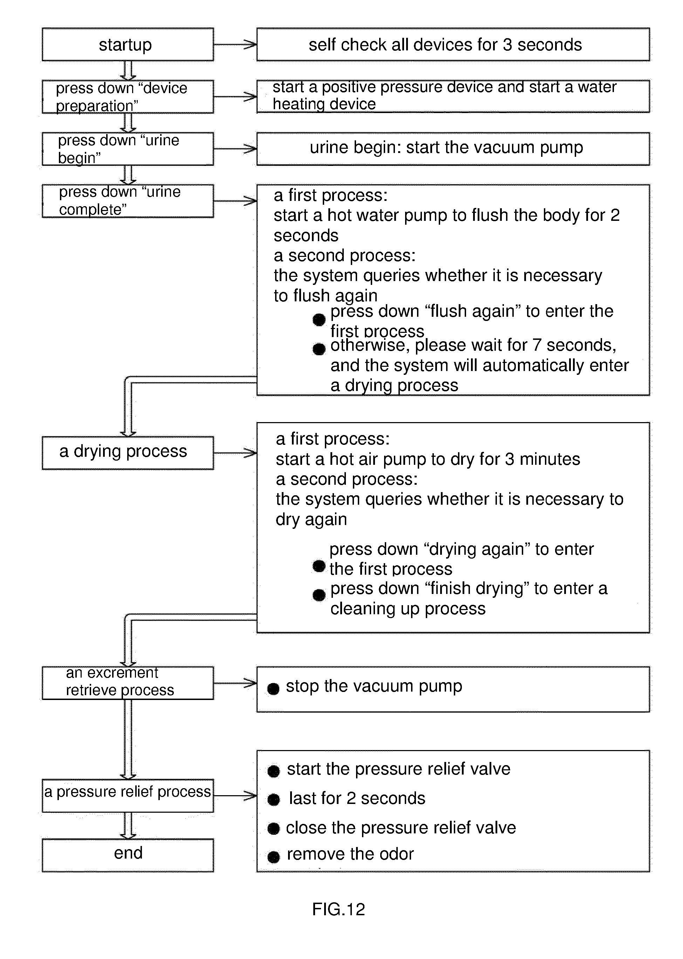

As shown in Fig. 12, in the wheelchair-type excrement automatic cleaning apparatus without need of undressing and dressing of the present invention, the urine process is realized as follow via the interactive unit 261 or a remote controller:
(1) after startup, self checking all of the above mentioned devices for 3 seconds;
(2) pressing down "device preparation" to start an air pump of the positive pressure device 23, i.e. a positive pressure pump, and to start a water heating device of the hot water device 24;
(3) pressing down "urine begin" to start the vacuum pump 221 of the negative pressure device 22;
(4) pressing down "urine complete" to enter a first process, in which a hot water pump of the hot water device 24 is started to flush the body for 2 seconds; and enter a second process, in which the system queries whether it is necessary to flush again, then enter the first process when pressing down "flush again", and otherwise, "please wait for 7 seconds", the system will automatically enter a drying process;
(5) a drying process, wherein in the first process, a hot air pump of the hot air device 25 is started to dry for 3 minutes, and then in the second process, in which the system queries whether it is necessary to dry again, then enter the first process when pressing down "drying again", or enter a cleaning up process when pressing down "finish drying";
(6) an excrement retrieve process, in which the vacuum pump 221 of the negative pressure device 22 is stopped; and
(7) a pressure relief process, in which the following actions are performed in sequence until the whole process is complete: starting the pressure relief valve, lasting for 2 seconds, closing the pressure relief valve, and removing the odor.

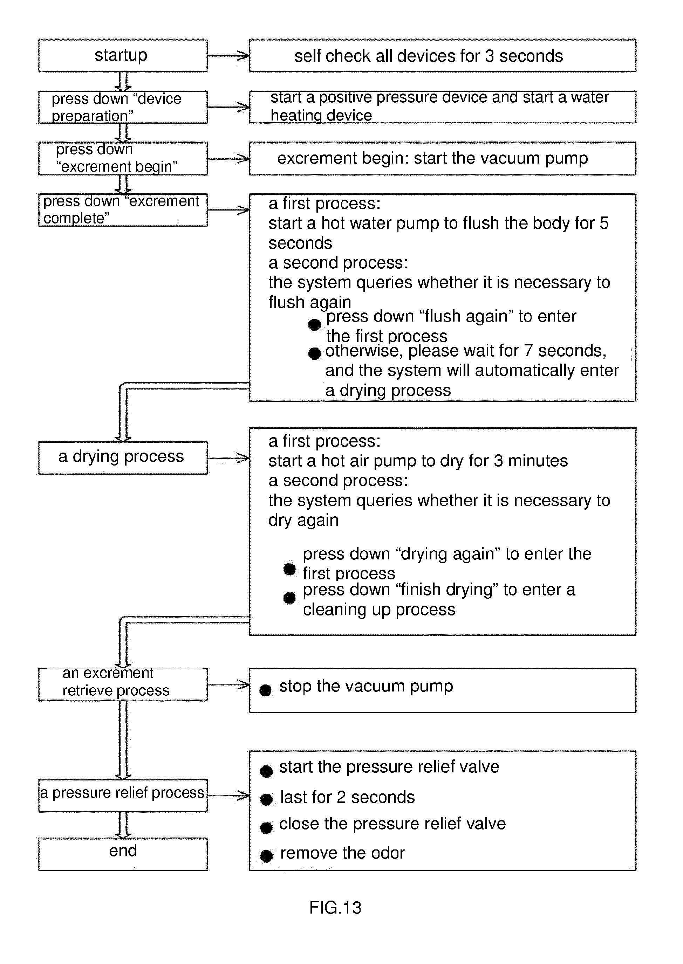

As shown in Fig. 13, in the wheelchair-type excrement automatic cleaning apparatus without need of undressing and dressing of the present invention, the excrement process is realized as follow:
(1) after startup, self checking all of the above mentioned devices for 3 seconds;
(2) pressing down "device preparation" to start an air pump of the positive pressure device 23, i.e., a positive pressure pump, and to start a water heating device of the hot water device 24;
(3) pressing down "excrement begin" to start the vacuum pump 221 of the negative pressure device 22;
(4) pressing down "excrement complete" to enter a first process, in which a hot water pump of the hot water device 24 is started to flush the body for 5 seconds; and enter a second process, in which the system queries whether it is necessary to flush again, then enter the first process when pressing down "flush again", and otherwise, "please wait for 7 seconds", the system will automatically enter a drying process;
(5) a drying process, wherein in the first process, a hot air pump of the hot air device 25 is started to dry for 3 minutes, and then in the second process, in which the system queries whether it is necessary to dry again, then enter the first process when pressing down "drying again", or enter a cleaning up process when pressing down "finish drying";
(6) an excrement retrieve process, in which the vacuum pump 221 of the negative pressure device 22 is stopped; and
(7) a pressure relief process, in which the following actions are performed in sequence until the whole process is complete: starting the pressure relief valve, lasting for 2 seconds, closing the pressure relief valve, and removing the odor.

The embodiments described above and the accompanying drawings do not intend to limit the form and style of the product according to the present invention, and the skilled in the art can make appropriate changes or modifications to the present invention without departing from the scope thereof.

## Claims

1. A wheelchair-type excrement automatic cleaning apparatus without need of undressing and dressing, **characterized in that**,
- it comprises a wheelchair, which is provided with a fully automatic disposal apparatus and self-adapting pants made of a soft facing material;
- the fully automatic disposal apparatus has an excretion duct, an excrement collecting device, a negative pressure device, a positive pressure device, a hot water device, a hot air device and a control device;
- the self-adapting pants are provided with an excrement collecting area at the crotch, and the excrement collecting area is provided with an excrement port corresponding to the discharging place of the human body, an inflating pipe, a hot water output pipe and a hot air output pipe;
- the excretion duct has an inlet end which is connected with the excrement port of the excrement collecting area, and an outlet end which is connected with the excrement collecting device, and the excrement collecting device is connected with the negative pressure device;
- the positive pressure device, the hot water device and the hot air device are correspondingly connected with the inflating pipe, the hot water output pipe and the hot air output pipe of the excrement collecting area via a pipe; and
- the control device controls the operation of the fully automatic disposal apparatus and the self-adapting pants.

2. The wheelchair-type excrement automatic cleaning apparatus without need of undressing and dressing according to claim 1, **characterized in that**,
- the cushion of the wheelchair is provided with an excrement port corresponding to the excrement port of the self-adapting pants;
- the negative pressure device, the positive pressure device, the hot water device and the hot air device are provided on the chair back of the wheelchair; and
- the excrement collecting device is provided below the cushion of the wheelchair.

3. The wheelchair-type excrement automatic cleaning apparatus without need of undressing and dressing according to claim 2, **characterized in that**,
- the excrement port of the cushion is provided with a cover which can open and close, and the control device controls the operation of the cover.

4. The wheelchair-type excrement automatic cleaning apparatus without need of undressing and dressing according to claim 1 or 2, **characterized in that**,
- the excrement collecting device is detachably connected with the wheelchair to form a removable connection.

5. The wheelchair-type excrement automatic cleaning apparatus without need of undressing and dressing according to claim 4, **characterized in that**,
- the excrement collecting device comprises an excrement container, the excrement container has an airtight hollow chamber, the hollow chamber is divided into an excrement collecting chamber and a negative pressure chamber by a partition plate, the partition plate is provided with a vent hole for communicating with the excrement collecting chamber and the negative pressure chamber;
- the excrement container is provided with openings, which correspond to the excrement collecting chamber and the negative pressure chamber and are arranged in a staggered manner;
- the opening of the excrement collecting chamber communicates with the outlet end of the excretion duct, and the opening of the negative pressure chamber communicates with the negative pressure device.

6. The wheelchair-type excrement automatic cleaning apparatus without need of undressing and dressing according to claim 1 or 2, **characterized in that**,
- the self-adapting pants are provided with a buttock bump at the rear inner surface of the excrement collecting area.

7. The wheelchair-type excrement automatic cleaning apparatus without need of undressing and dressing according to claim 6, **characterized in that**,
- the self-adapting pants are provided with a convex tube at the front outer surface of the excrement collecting area, and the inflating pipe, the hot water output pipe and the hot air output pipe are arranged in the convex tube, respectively.

8. The wheelchair-type excrement automatic cleaning apparatus without need of undressing and dressing according to claim 7, **characterized in that**,
- the self-adapting pants are provided with compatible sticking parts on the sides, and the excrement port of the self-adapting pants is provided with a quick connector compatible with the inlet end of the excretion duct.

9. The wheelchair-type excrement automatic cleaning apparatus without need of undressing and dressing according to claim 8, **characterized in that**,
- the waistline and nozzle of self-adapting pants are provided with a pneumatic sealing ring, respectively, and the pneumatic sealing ring is connected with an inflating device via a pipe.

10. The wheelchair-type excrement automatic cleaning apparatus without need of undressing and dressing according to claim 1 or 3, **characterized in that**,
- the control device comprises an interactive unit which is connected with the wheelchair in a flip and folding manner to form a removable connection.
